# EUROPEAN PATENT SPECIFICATION

(11) **EP 4 023 176 B1**
(45) Date of publication and mention of the grant of the patent: **31.12.2025**
(21) Application number: 21196949.8
(22) Date of filing: 15.09.2021
(51) Int. Cl.: A61B 17/34

(54) **A STABILISER**
STABILISATOR
STABILISATEUR

(30) Priority: 21.09.2020 US 202017027561
(43) Date of publication of application: 06.07.2022
(73) Proprietor: The Provost, Fellows, Scholars and other Members of Board of Trinity College Dublin, Dublin 2 (IE)
(72) Inventor: Ryan, Garrett, Co. Tipperary (IE); McGarvey, Colm, Dublin 4 (IE)
(74) Representative: Purdylucey Intellectual Property

(56) References cited:
- WO-A1-2019/180154
- US-A- 5 263 939
- US-A1- 2019 254 703

## Description

### Field of the Invention

This invention relates to a stabilizer and more particularly to a medical stabilizer for medical devices such as needles and the like.

### Background to the Invention

Many medical procedures require the accurate positioning, alignment and stabilizing of devices and instruments such as needles, catheters, drains, surgical pins, screws, drains and tubes during medical procedures performed on a patient.

For example, in computed tomography (CT) and fluoroscopic guided procedures, X-ray imaging is employed to guide a needle to a target in a patient. In order to position the needle at the target, a clinician must align the needle axis with the X-ray beam and advance the needle in the patient whilst taking a number of CT or fluoroscopic images which inform the movement of the needle. This process is repeated until the needle is positioned at the desired target. In such procedures, it is important that the position of the needle is maintained and stable while the CT images are being taken e.g. if the needle does not have sufficient traction within the patient's tissue, or if the weight of the proximal end of the needle is excessive, the needle can sag once released and lose its orientation. Patient movement can also result in undesired reorientation of the needle. This undesired movement of the needle results in additional X-ray images being required to correctly reposition the needle so that the clinician and the patient are exposed to increased potentially harmful radiation levels.

Various guide devices have been developed to assist clinicians in guiding and positioning needles and the like during medical procedures. For example, PCT Patent Specification No. 2016/186937 and EP 3 150 150 both describe a needle guide device having a non-resilient slotted sphere defining a guide channel for a needle in which the non-resilient sphere is locked in position by tightening a threaded ring surrounding the stiff slotted sphere to close the slot and tightly grip the needle. Similarly, US Patent Specification No. 3,021,842 describes a hypodermic needle guide in which a solid ball having a needle passage for guiding the needle can be locked in position with a threaded locking ring. However, the spheres/balls employed in such guide devices simply act as guides for the needle and do not grip the needle passing through the sphere/ball whilst the stiff slotted spheres in combination with the threaded tightening mechanisms lack sensitivity when gripping and controlling movement of the sphere/ball i.e. needles are either tightly gripped in by the sphere when the slot is closed or are free to move unhindered in an uncontrolled manner in the sphere with the slot is open so that controlled and fine movement of the needle in the sphere is not possible. More generally, such devices and other known devices, being formed of multiple components are difficult and expensive to manufacture whilst still failing to allow for fine and accurate adjustment of a locking, gripping and stabilizing force on the needle.

WO 2019/180154 A1 describes a medical device stabilizer in which the medical device grip is in the form of a compressible ball attached to a base member. US 5 263 939 A describes a retainer for a laparoscopic cannula provided with a compressible plug attached to a clamp. US 2019/254703 A1 describes a port fixation device made up of a body portion and a plug engaged with the body portion.

### Summary of the Invention

The invention is set out in the appended set of claims.

Accordingly, there is provided a medical device stabilizer comprising
a base member;
a medical device grip comprising a ball mountable on a medical device in a frictionally slidable relationship and positionable on the medical device to select a desired medical device insertion depth, and defining a medical device guide bore through the medical device stabilizer, and
a coupler in the form of a hinged clamping mechanism on the base member for engaging the medical device grip, the coupler being movable between a medical device grip engaged position and a medical device grip release position wherein the medical device stabilizer is a two-part medical device stabilizer in which the medical device grip is separate and independent of the base member but is engageable with and detachable from the base member at the coupler and wherein the medical device guide bore is configured to form a slidable friction fit with a medical device and hold the medical device grip in a desired position on the medical device without the base member in use.

Suitably, the medical device grip comprises a resilient material.

Preferably, the resilient material has a durometer reading of from about 20A to about 90A.

More preferably, the resilient material has a durometer reading of from about 40A to about 80A. Most preferably, the resilient material has a durometer reading of about 70A.

Advantageously, the coupler comprises a clamping mechanism. Preferably, the clamping mechanism comprises a hinged clamp mechanism.

Suitably, the clamping mechanism comprises a collet defining a socket for the medical device grip. Preferably, the collet is formed around a central opening formed in the base member.

Preferably, the collet serves as a hinge of the clamping mechanism.

Suitably, the clamping mechanism comprises at least one fingergrip to effect a ball clamping action. Advantageously, the clamping mechanism comprises a pair of pinchable fingergrips operable to hingedly open the socket to receive a medical device grip and hingedly close the socket to clamp the medical device grip.

In one embodiment, the fingergrips are formed on a top face of the base member and are spaced apart by a V-shaped indent in the base member.

Suitably, the base member comprises an adhesive layer for adhering the medical device stabilizer to a patient.

Advantageously, the guide bore passes through the centre of the medical device grip.

The medical device grip can be provided with grip indents on an outer surface of the medical device grip.

The medical device stabilizer can comprise a compression mechanism to compress the medical device grip.

The compression mechanism can comprise a compression hinge. The compression mechanism can comprise a pair of compression hinges.

Suitably, the pair of compression hinges are hingedly openable and closable by fingergrips extending from the compression hinges.

The medical device stabilizer can further comprise a locking mechanism to maintain a compression force on the medical device grip. Preferably, the locking mechanism comprises a living hinge.

While not specifically claimed also described is a medical device stabilizer system comprising a medical device stabilizer and a medical device wherein the medical device stabilizer comprises
a base member;
a medical device grip, engageable with the base member, defining a medical device guide bore through the medical device stabilizer, the medical device guide bore being configured to form a slidable friction fit with the medical device, and
a coupler on the base member for engaging the medical device grip, the coupler being movable between a medical device grip engaged position and a medical device grip release position.

The medical device can be a needle.

While not specifically claimed also described is a method of securing and positioning a medical device with respect to a subject, the method comprising the steps of:
employing a medical device stabiliser having
   a base member;
   a medical device grip, engageable with the base member, defining a medical device guide bore through the medical device stabilizer, the medical device guide bore being configured to form a slidable friction fit with a medical device, and
   a coupler on the base member for engaging the medical device grip, the coupler being movable between a medical device grip engaged position and a medical device grip release position;
placing a medical device through the medical device guide bore of the medical device grip;
inserting the medical device into the subject, and
engaging the medical device grip with the coupler to stabilize the medical device.

The medical device is inserted into the subject to a predetermined depth determined by the location of the medical device grip on the medical device.

The present invention therefore provides a device for positioning and securing a medical device, instrument or other piece of equipment. It is particularly suited for securing needles, particularly coaxial needles during image guided procedures.

In summary, the stabilizer of the invention has a simple and effective design which is easy and economical to produce; can be produced as a low-cost disposable item; is lightweight and compact and is ergonomic and easy to use whilst being easily adjustable for repositioning of medical devices such as needles. The stabilizer allows for effective needle grip, support and orientation and can be used for a range of medical and surgical procedures including biopsy, drainage, injection and ablation. The stabilizer of the invention is especially efficacious for difficult angulation and positioning procedures involving difficult targets - especially for targets that are small and deep inside the body - so that procedure lengths and X-ray exposure for patients and clinicians alike are reduced significantly. Due to its ease of use, the stabilizer of the invention is suitable for use in hospitals, ambulatory centres, diagnostic centres, surgical and ablation centres.

### Brief Description of the Figures

The invention will now be described, by way of example only, with reference to the accompanying drawings and non-limiting Example in which:
Figure 1 is a perspective view from above and one side of a medical stabilizer not according to the claimed invention having a resilient medical device grip in the form of a conformable or compressible ball formed from a compressible resilient material and a ball compression mechanism made up of an outer tightening nut defining an oval opening for receiving an oval collet surrounding the conformable ball so that rotation of the tightening nut causes the collet to compress the ball, the tightening nut being mounted on the collet at a snap-fit mounting;
Figure 2 is a top plan view of the medical stabilizer of Figure 1;
Figure 3 is a cross-sectional view along the line III-III of Figure 2;
Figure 4 is a cross-sectional view along the line IV-IV of Figure 3;
Figure 5 is a cross-sectional view along the line V-V of Figure 3 with the letter P indicating the large diameter of the oval opening in the tightening nut and the oval collet and the letter Q indicating the small diameter of the oval opening in the tightening nut and the oval collet;
Figure 6 is an exploded perspective view from above and one side of the medical stabilizer of Figure 1;
Figure 7 is a schematic side elevation of the medical stabilizer of Figure 1 positioned on a patient with a CT-guided needle inserted in the guide bore of the ball of the stabilizer with the needle in a vertical position oriented towards its target;
Figure 8 is a schematic side elevation of the medical stabilizer of Figure 1 positioned on a patient with a CT-guided needle inserted in the guide bore of the ball of the stabilizer with the ball rotated in the socket so that the needle is articulated offset from the vertical and oriented towards its target;
Figure 9 is an enlarged cross-sectional view, similar to the cross-sectional view of Figure 5, of a example of a medical device stabiliser not according to the claimed invention in which the conformable ball is provided with slits arranged in a cruciform pattern to increase the compressibility of the ball;
Figure 10 is a perspective view from above and one side of a third example of a medical device stabiliser not according to the claimed invention in which the conformable ball is provided with an extension for receiving and supporting a medical device such as a needle;
Figure 11 is a perspective view from above and one side of a fourth example of a medical device stabiliser in which the medical stabilizer is provided with accessory mountings for attaching accessories to the stabilizer;
Figure 12 is a perspective view from above and one side of a fifth example of a medical device stabiliser holding a needle in which the medical stabilizer includes a fixing plate mountable over and co-operable with the base member of the medical stabilizer to fix the medical stabilizer in position on a patient;
Figure 13 is a perspective view from above and one side of a sixth example of a medical device stabiliser in which the base member in the form of a base plate of the medical stabilizer is segmented into flexible petal-like members to enhance contouring of the base plate to a patient's skin;
Figure 14 is a perspective view from above and one side of a seventh example of a medical device stabiliser in which the tightening nut is mounted on the collet via outwardly extending fingers on the collet inserted in horizontal slits defined in the tightening nut;
Figure 15 is a top plan view of the medical stabilizer of Figure 14;
Figure 16 is a side elevation of the medical stabilizer;
Figure 17 is an exploded perspective view from above and one side of the medical stabilizer;
Figure 18 is an exploded perspective view from above and one side of an eighth example of the medical stabilizer in which the actuator mounting is made up of an outer threaded tightening nut and a complementary threaded collet;
Figure 19 is a cross-sectional view through the medical stabilizer of Figure 18 showing the internal threads of the tightening nut;
Figure 20 is a perspective view from above and one side of the medical stabilizer of Figure 18 with the threaded nut secured to the threaded collet;
Figure 21 is a cross-sectional view through the medical stabilizer of Figure 20;
Figure 22 is an exploded perspective view from above and one side of a ninth example of the medical stabilizer device in which the grip compression mechanism is made up of a band clamp type mechanism;
Figure 23 is a perspective view from above and one side of the medical stabilizer of Figure 22 with the band clamp type mechanism partially closed,
Figure 24 is a perspective view from above and one side of the medical stabilizer with the band clamp type mechanism fully closed;
Figure 25 is a perspective view from above and one side of another example of a medical stabilizer;
Figure 26 is a perspective view from above and one side of an embodiment of the medical device stabilizer system of the invention in which the medical device stabilizer is a two-part stabilizer made up of the medical device grip and, separately, the base member with the medical device grip being slidably mountable on a medical device such as a coaxial delivery needle as shown and being engageable with and detachable from the base member at a coupling/clamping mechanism;
Figure 27 is an exploded perspective view from above and one side of the base member, skin adhesive tape and backing tab;
Figure 28 is a top plan view of the base member and a cross-sectional view along the line A-A of the base member;
Figure 29 is a perspective view from above and one side of the medical device grip mounted on the coaxial delivery needle which is inserted a patient body part;
Figure 30 is a perspective view from above and one side of the medical device grip and coaxial delivery needle of Figure 29 with the base member approaching the medical device grip to clamp the medical device grip via the coupling mechanism with the direction of approach and clamping action indicated by the arrows;
Figure 31 is a perspective view from above and one side of the coaxial delivery needle inserted in the patient body part with the medical device grip clamped by the coupling mechanism to hold the coaxial delivery needle in position on the patient, and
Figure 32 is a top plan view of the base member of a further embodiment of the invention in which the base member of Figures 26 to 31 is also provided with a compression mechanism to compress the medical device grip and firmly lock medical devices in position in the medical device grip.

### Detailed Description of the Invention

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

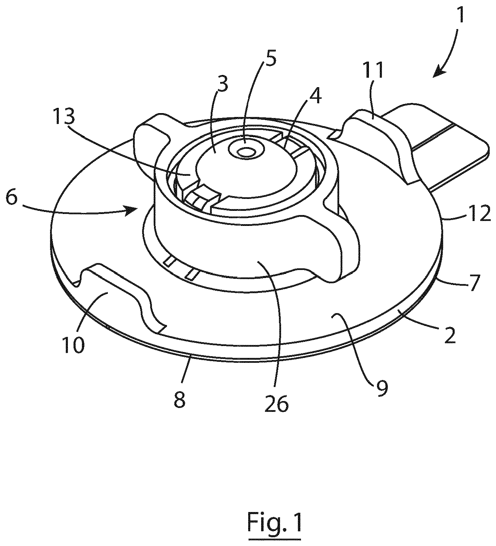

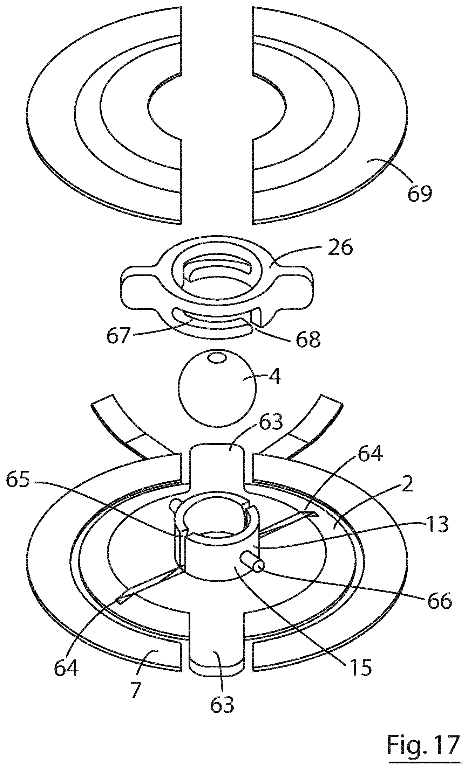

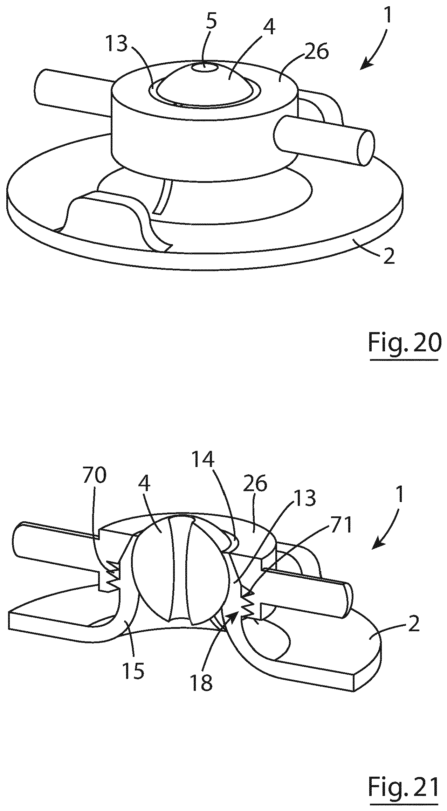

While not specifically claimed, Figures 1 to 25 describe a medical device stabilizer generally indicated by the reference numeral 1 made up of a base member in the form of a substantially circular base plate 2 and a compressible medical device grip 3 on the base plate 2 which can articulate for gripping and positioning medical devices such as CT-guided needles held in the medical device grip 3. The base plate is circular, but it may be a different shape such as square, rectangular or oval. The medical device grip 3 is in the form of a resilient and compressible ball 4 formed from a compressible resilient material having a guide bore 5 therethrough for receiving and holding the medical devices with the degree of compression of the ball 4, and hence the grip on medical devices in the guide bore 5, being controllable by a grip compression mechanism 6 surrounding the ball 4 which is movable between a ball compressed position and a ball non-compressed position. The guide bore 5 is sized so that resilient and conformable material of the compressible ball maintains a constant friction-fit type contact with medical devices inserted in the guide bore 5. The medical device stabilizer 1 can be secured to a patient via an adhesive e.g. in the form of an adhesive layer or tape 7 provided on the base plate 2.

Figures 1 to 8 show a first medical device stabilizer 1. Referring specifically to Figures 1 to 5, the circular base plate 2 has a base plate bottom face 8 and a base plate top face 9 provided with two peripheral oppositely disposed first and second finger grips 10,11 respectively at the base plate edge 12 for holding and positioning the base plate 2.

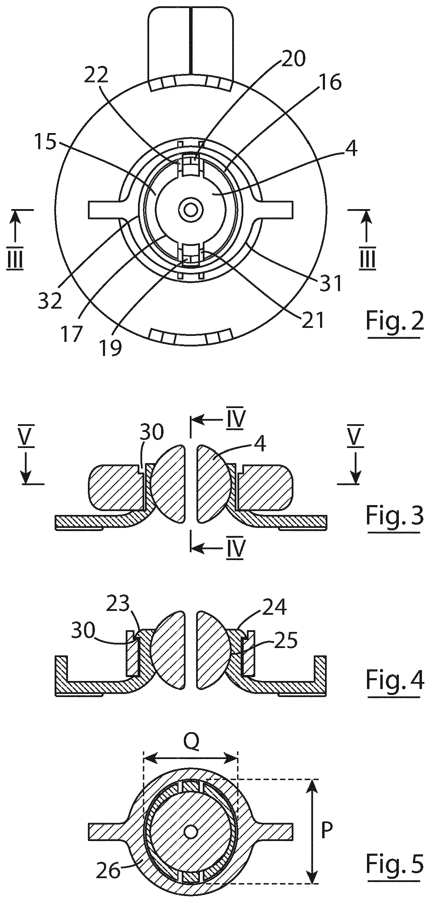

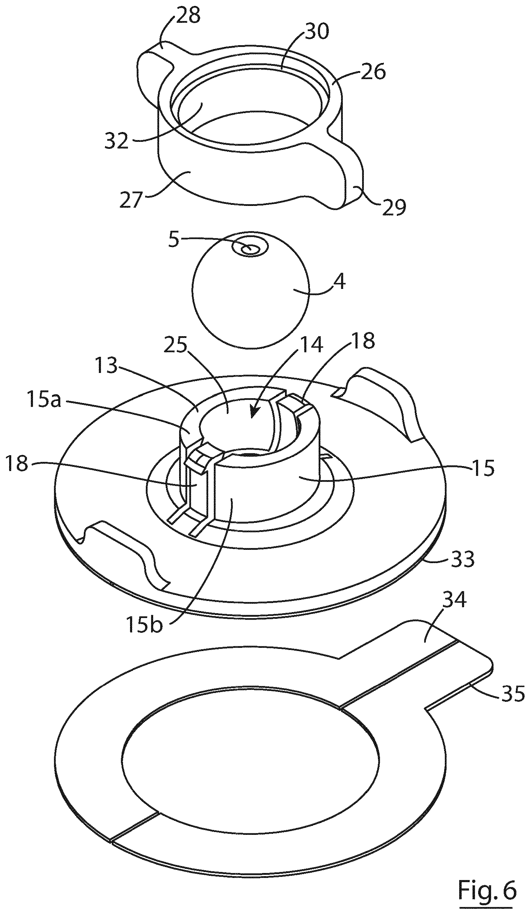

The grip compression mechanism 6 is a two-part mechanism made up firstly of a collet 13 centrally formed on the base plate 2. The collet 13 is integrally formed by the base plate 2 so that the collet 13 and base plate 2 constitute a unitary piece which can be injected moulded if desired. Referring to Figure 6, the collet 13 has a spherical socket 14 (complementary in size and shape with the compressible ball 4) for receiving and holding the compressible ball 4 defined by a flexible collet wall 15 upstanding from the base plate 2. The collet wall 15 has an oval outer profile 16 when viewed from above and a circular inner profile 17 when viewed from above defining the circular socket 14 (see in particular Figures 2 and 5). The flexible collet wall 15 allows for insertion and retention of the compressible ball 4 in the socket 14 in a press-fit or snap-fit fitting whilst allowing the compressible ball 4 to rotate in the socket 14 i.e. internally, the collet wall 15 is shaped and contoured as indicated by the reference numeral 25 in Figure 6 so that the socket 14 complements the spherical shape of the ball 4. Accordingly, the flexible collet wall 15 can flex laterally outwards to receive the compressible ball 4 in the socket 14 and then return inwards via the press-fit or snap-fit action to hold the compressible ball 4 in place.

Referring to Figure 2, the collet wall 15 is segmented by first and second oppositely disposed first and second flexible ribs 19,20 defined in the collet wall 15. The ribs 19,20 are each formed by respective first and second pairs of slots 21,22 respectively defined in the collet wall 15 so that the flexible collet wall 15 is made up of the oppositely disposed flexible ribs 19,20 and oppositely disposed flexible wall segments 15a,15b (Figure 6). At their free ends, the flexible ribs 19,20 have respective first and second catches 23,24 (Figure 4).

Referring to Figure 6, the second part of the two-part grip compression mechanism 6 is made up of a manual actuator in the form of a ring-like tightening nut 26 sized and shaped to surround the collet 13. The tightening nut 26 has an annular wall 27 provided with first and second oppositely disposed external wings 28,29 respectively for turning the tightening nut 26 about the collet 13. Internally, the tightening nut 26 has an annular flange 30 for engaging the first and second catches 23,24 on the flexible ribs 19,20. The tightening nut 26 is sized and shaped to fit over the collet 13 in a snap-fit relationship i.e. the first and second catches 23,24 and the annular flange 30 in combination define a snap-fit actuator mounting 18 between the collet 13 and the tightening nut 26. The tightening nut 26 can be rotated or turned about the collet 13 in the actuator mounting 18.

Referring to Figures 2 and 6, the wall 27 of the tightening nut 26 has a circular outer profile 31 when viewed from above and an oval inner profile 32 when viewed from above which complements the oval outer profile 16 of the collet 13. Accordingly, as shall be explained more fully below, the grip compression mechanism 6 is an offset eccentric circle tightening mechanism in which the tightening nut wall 27 interferes with the collet wall 15 during clockwise and anti-clockwise rotation to displace the collet wall segments 15a,15b and the flexible ribs 19,20 inwards and compress the compressible ball 4.

The adhesive layer 7 of the medical device stabilizer 1 is made up of a circular adhesive tape 33 attached to the bottom face 8 of the base plate 2. Removable backing tabs 34 and 35 are provided to protect the circular adhesive tape 33 prior to use of the stabilizer device 1.

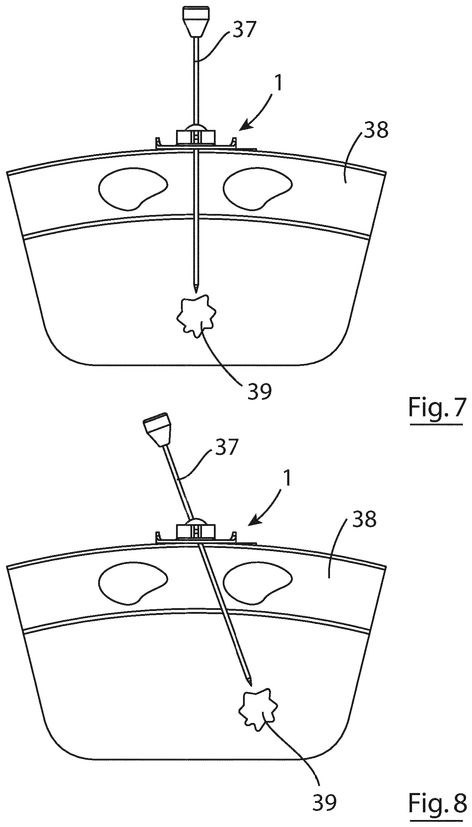

In use, the stabilizer device 1 can be used with a range of medical devices and in a variety of procedures such as biopsy, drainage, injection and ablation procedures. Figures 7 and 8 show the stabilizer device 1 in use with a CT-guided needle 37 which is inserted through the guide bore 5 of the ball 4 so that stabilizer device 1 is located on the CT-guided needle 37. If desired, the diameter of the guide bore 5 can be sized so that CT-guided needle 37 enjoys a friction fit in the guide bore 5 to hold the stabilizer device 1 on the CT-guided needle 37.

The CT-guided needle 37 is then inserted into a patient body part and, following removal of the removable backing tabs 34 and 35 from the adhesive tape 33, the base plate 2 is positioned as required on the patient body part 38 adjacent a desired target 39 in the body part 38. Under X-ray guidance, the CT-guided needle 37 can be positioned as required via the rotatable and articulatable ball 4 which can be compressed by the grip compression mechanism 6 as previously described when required to hold, fix and stabilise the CT-guided needle 37 in a desired position in the stabilizer 1. More particularly, as the resilient and conformable material of the spherical ball 4 is in constant friction-fit type contact with CT-guided needle 37, the CT-guided needle 37 is constantly supported in place in medical device stabilizer 1 so that no undesirable translational or sliding movement of the CT-guided needle 37 occurs i.e. the CT-guided needle 37 must be deliberately slid in the guide bore 5 by a user to effect movement of the CT-guided needle 37. This in contradistinction with devices of the prior art where needles and the like are supported in rigid materials which either fully grip the needles preventing all sliding movement or allow full sliding movement of the needles resulting in poor sensitivity and movement control in use.

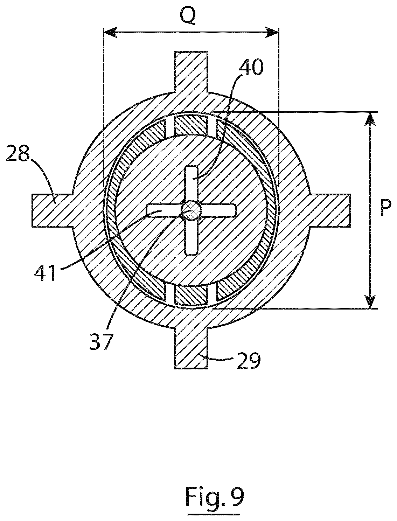

Figure 9 is an enlarged cross-sectional view, similar to the cross-sectional view of Figure 5, of a second stabilizer 1 that is broadly similar to the stabilizer 1 of Figures 1 to 8 and in which parts the same as those previously described are assigned the same reference numerals. However, the compressible ball 4 is provided with slits 40 to increase the compressibility of the ball 4 around the CT-guided needle 37. The slits 40 extend through the compressible ball 4 and are arranged in a cruciform pattern 41 about the guide bore 5. The tightening nut 26 is also provided with four peripheral wings 28,29.

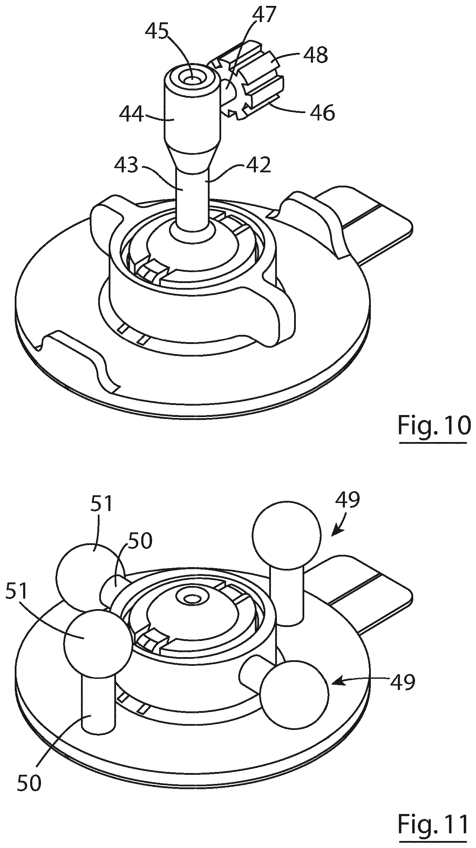

Figure 10 shows a perspective view from above and one side of a third stabilizer 1 also broadly similar to the stabilizer 1 of Figures 1 to 8 and like numerals indicate like parts. The stabilizer 1 is provided with a detachable medical device auxiliary extension piece 42 insertable in and removable from the guide bore 5 to provide increased support for medical devices such as CT-guided needles 37. The extension piece 42 is made up of a tube 43 insertable in the guide bore 5 at its first end and a cylindrical neck 44 at its opposite end for receiving a medical device in a neck opening 45 defined in the neck 44. The extension piece 42 is provided with a locking system 46 to secure a medical device in position in the extension piece 42. The locking system 46 is made up of a screw shaft 47 insertable into the cylindrical neck by rotating a head 48 on the shaft 47 so that the screw shaft 47 is placed into frictional engagement with a medical device positioned in the cylindrical neck 44.

Figure 11 shows a perspective view from above and one side of a fourth stabilizer 1 also broadly similar to the stabilizer 1 of Figures 1 to 8 and like numerals indicate like parts. However, the stabilizer 1 is provided with accessory mountings 49 for attaching accessories to the stabilizer 1 as required. Two oppositely disposed accessory mountings 49 extend upwards from the top face 9 of the base plate 2 either side of the grip compression mechanism 6 while the first and second wings 28,29 of the tightening nut 26 are also replaced by similar accessory mountings 49 extending laterally outwards from the tightening nut wall 27. Each accessory mounting 49 is made up of a shaft 50 attached at one end to the base plate 2 or the tightening nut wall 27 and a spherical mounting head 51 at its opposite end. However, the accessory mountings 49 can be shaped and dimensioned as required.

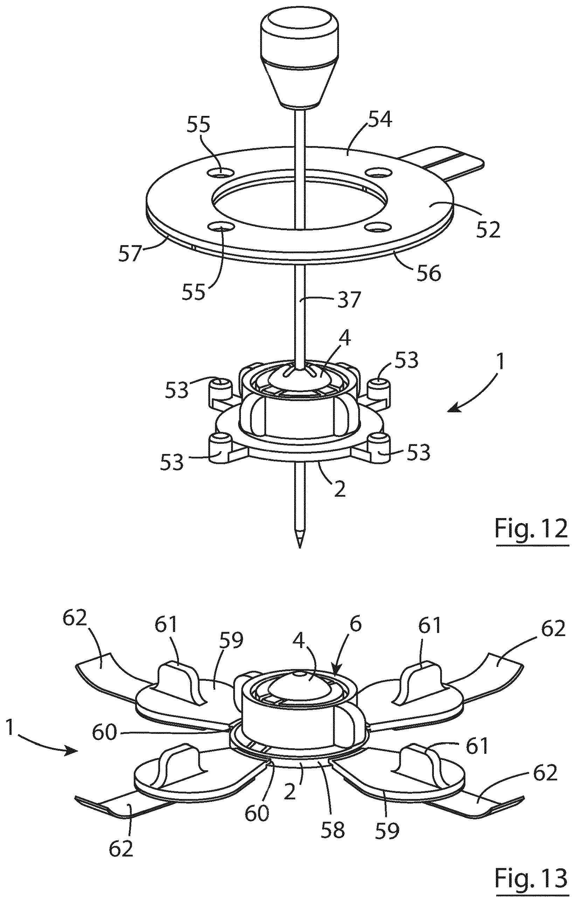

Figure 12 shows a perspective view from above and one side of a fifth stabilizer 1 of the invention similar to the stabilizer 1 of Figure 9 with a CT-guided needle 37 gripped in the ball 4 of the stabilizer 1. Like numerals indicate like parts. However, the stabilizer 1 is provided with a fixing plate 52 mountable over and co-operable with the base plate 2 of the medical stabilizer 1 to assist in fixing the medical stabilizer 1 in position on a patient. More particularly, base plate 2 has four spaced-apart peripheral upstanding fixing lugs 53 for engagement with the fixing plate 52 which has an annular fixing ring 54 provided with spaced apart lug holes 55 for receiving the fixing lugs 53 on the base plate 2. In use, the fixing plate 52 is placed over the base plate 2 so that the fixing lugs 53 are received in the lug holes 55 while the annular fixing ring 54 is provided with an adhesive layer 56 to adhere the annular ring 54 to a patient. The adhesive layer 56 is protected by a removable backing 57 prior to use.

Figure 13 shows a perspective view from above and one side of a sixth stabilizer 1 similar to the stabilizer 1 of Figures 1 to 8. Like numerals indicate like parts. The base plate 2 of the medical stabilizer 1 is segmented so that base plate 2 can flex to meet contours on a patient. More particularly, the base plate 2 is formed from a central disc 58 on which the conformable ball 4 and grip compression mechanism 6 are mounted as previously described. However, four spaced apart petal-like base plate segments 59 extend laterally outwards from the central disc 58 at flexible joints 60 defined between the base plate segments 59 and the central disc 58 so that each base plate segment 59 can flex and bend independently as required in accordance with a patient's surface contours. Each base plate segment 59 is provided with a finger hold 61 on its upper surface while adhesive tabs 62 are provided on the lower surface of each base plate segment 59 to adhere the base plate segments 59 to patients.

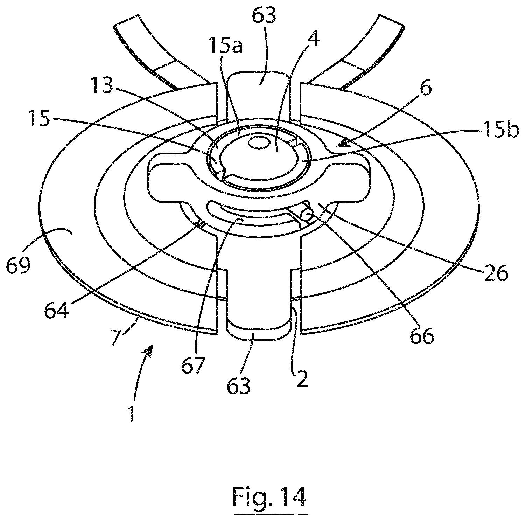

Figures 14 to 17 show a seventh stabilizer 1 similar in function to the medical stabilizer 1 of Figures 1 to 13 but in which the snap fit actuator mounting 18 is replaced by an alternative actuator mounting. Like numerals indicate like parts.

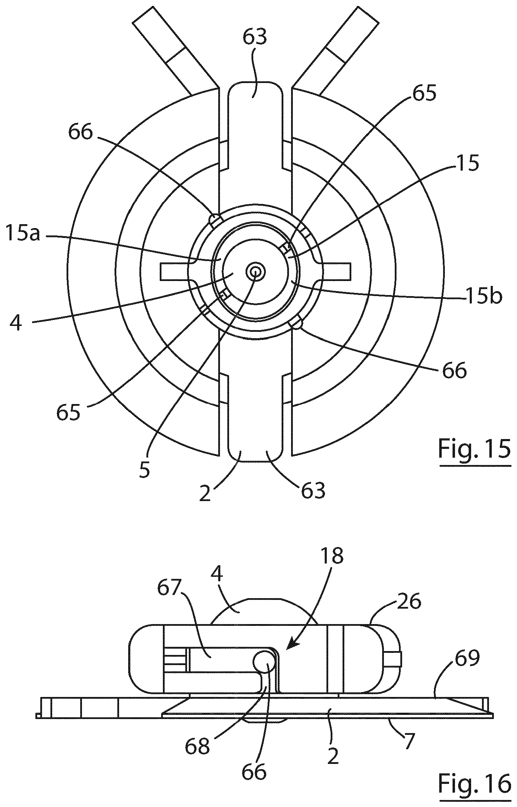

As shown in the drawings, the base plate 2 is provided with peripheral laterally extending and oppositely disposed tags 63 to facilitate contouring of the base plate 2 against a patient and gripping of the base plate 2. In addition, flexing of the base plate 2 is enhanced by a channel 64 defined in the base plate 2 which extends across the base plate 2. The grip compression mechanism 6 is made up of a collet 13 and an annular tightening nut 26 having oval outer and inner profiles when viewed from above respectively as previously described (see Figure 15). However, the collet 13 has two oppositely disposed slots 65 to define the oppositely disposed flexing collet wall segments 15a,15b. Externally, the collet wall 15 is provided with oppositely disposed outwardly extending fingers 66 for insertion in complementary substantially horizontal slits 67 defined in the tightening nut 26 so that the tightening nut 26 is mounted on the fingers 66 and can also be rotated about the collet 13 on the fingers 66 along a path defined by the slits 67. The fingers 66 are received in the slits 67 via slit mouths 68 defined in the tightening nut 26. The fingers 66 on the collet wall 15 and the complementary slits 67 therefore form the actuator mounting 18.

The stabilizer 1 is further provided with an annular base plate cover 69 with an adhesive layer 7 to further assist in securing the medical stabilizer to a patient.

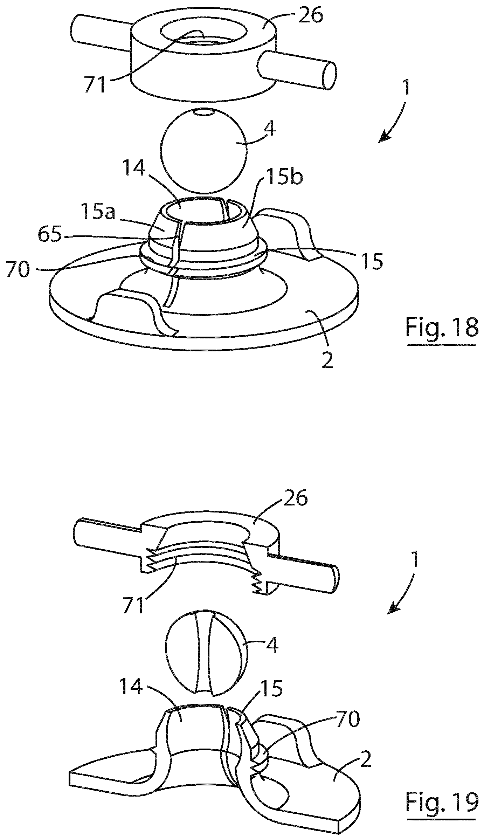

Figures 18 to 21 show an eighth medical stabilizer 1 broadly similar to the previous medical stabilizers and like numerals indicate like parts. However, , the collet wall 15 is provided with two oppositely disposed slots 65 to define two wall segments 15a,15b similar to the collet 13 of Figures 14 to 19 and is further provided with a collet screw thread 70 on the collet wall 15 engageable with a complementary screw thread 71 defined on the internal face of the tightening nut 26. The collet 13 and the tightening nut 26 have circular profiles when viewed from above while the collet wall 15 is inclined inwards to retain the compressible ball 4 in the socket 14 defined by the collet 13. The external wall of the wall segments 15a, 15b is chamfered to correspond to a similarly chamfered internal wall of the tightening nut 26. The screw threads 70,71 therefore form the actuator mounting 18 while rotation of the tightening nut 26 on the collet 13 causes the chamfered wall segments 15a,15b to flex and compress the compressible ball 4.

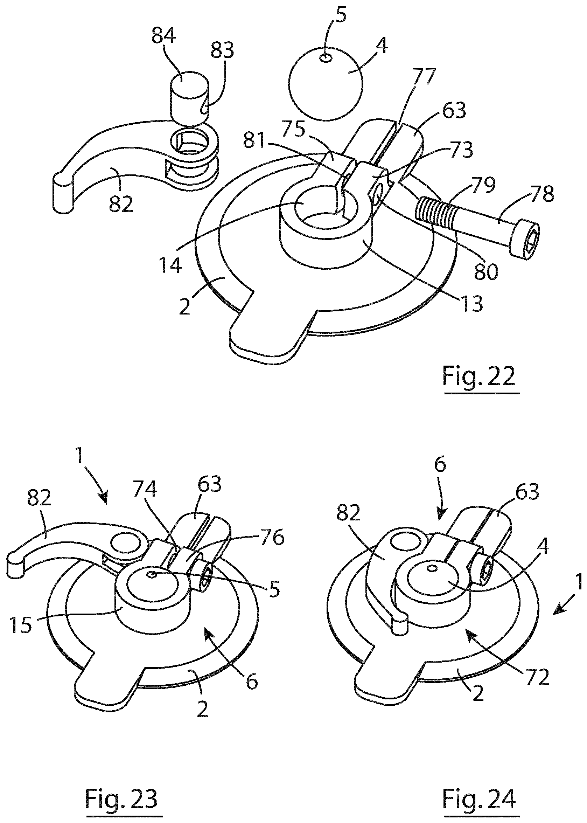

Figures 22 to 24 show a ninth medical stabilizer device 1 similar to the stabilizer devices 1 previously described but in which the grip compression mechanism 6 is a band clamp 72 and the tightening nut 26 is omitted. Like numerals indicate like parts.

As shown in the drawings, , the collet wall 15 of the collet 13, which forms the band of the band clamp 72, is provided with a mounting block 73 to one side of the collet wall 15. A single mounting block slit 74 is defined in the mounting block 73 to form a handle mounting portion 75 and a screw mounting portion 76 separated by the mounting block slit 74. The collet wall 15 defines a socket 14 for the compressible ball 4 which can be compressed by urging the handle mounting portion 75 towards the screw mounting portion 76 to close the slit 74. In addition, the base plate 2 is provided with a base plate slit 77 contiguous with the mounting block slit 74 which closes with the mounting block slit 74.

A screw 78 with a screw thread 79 is held in the screw mounting portion 76 at a first screw hole 80 and extends through a similar second screw hole 81 in the handle mounting portion 75 to engage an arcuate handle 82 at a threaded opening 83 defined in an insert 84 supported in the handle 82. Movement of the arcuate handle 82 between an open position as shown in Figure 23 and a closed position shown in Figure 24 where the arcuate handle 82 abuts the collet wall 15 exerts a pulling action on the screw 78 to close the mounting block slit 74 and the base plate slit 77 so that the collet wall 15 clamps and compresses the compressible ball 4 to grip a medical device such as a CT-guided needle 37 (not shown) in the guide bore 5 of the compressible ball 4.

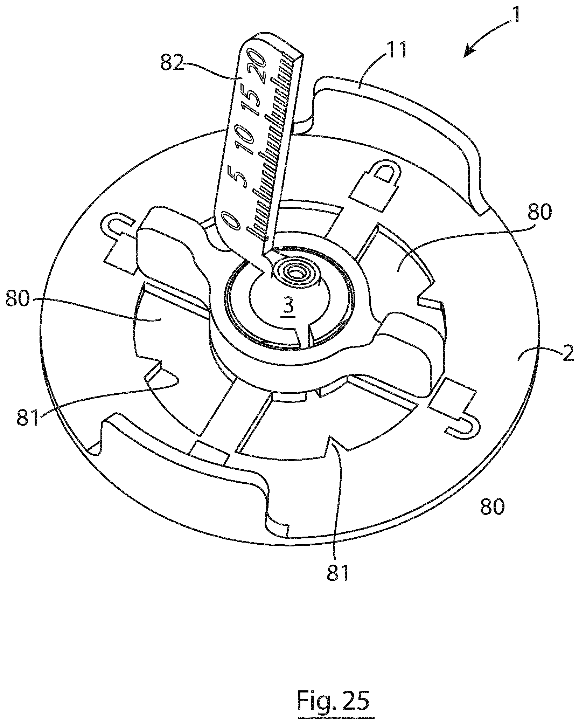

Figure 25 shows a tenth medical stabiliser device 1 in which parts described with reference to the previous medical stabilizers are assigned the same reference numerals. The circular base plate 2 includes four arcuate windows 80 for the purpose of facilitating the placement of the device on a target area of the body. An outer circumference of each window 80 includes a triangular projection 81 pointing towards the centre of the device. In addition, the compressible ball 4 includes a ruler 82 that projects upwardly from the ball, and is used to accurately target the depth of insertion of a medical device though the guide bore 5 during use.

### Example

A device as shown in Fig. 1 - Fig. 6 was created to evaluate different ball materials and durometers. The base plate 2 and tightening nut 26 were created from polylactic acid (PLA) using an Ultimaker 2 rapid prototyping machine. In the present Example, a selection of balls of 3/8" diameter and of different materials and durometers were employed. The molded ball was formed by making a 3/8" spherical mold and injecting it with degassed silicone (Dragon Skin FX-Pro, Smooth-On Inc., USA). A central guide bore of approx. 1.5mm diameter was cored out of each ball using a coring drill bit so that each ball fitted snugly, with a friction fit, on to a 17Gauge needle (OD approx. 1.5mm). The guide bore was slightly conical in profile - being slightly wider on the needle entry side compared to the needle exit side. Each ball was inserted into the device assembly and the assembly was evaluated in bench testing. The same base plate, needle and tightening nut were used in each case and the balls were swapped in and out. Each assembly was evaluated according to the attributes as outlined in Table 1.

**Table 1. Device description and evaluation of device performance in relation to the ball material and durometer**

| | **Device 1** | **Device 2** | **Device 3** | **Device 4** |
|---|---|---|---|---|
| **Ball Material:** | Silicone | Nitrile | Viton | Polyurethane |
| **Ball Durometer:** | 2A | 70A | 70A | 95A |
| **Vendor/Supplier** | Molded | The Precision Plastic Ball Co. Ltd (UK) | Dejay Distribution Ltd (UK) | Dejay Distribution Ltd (UK) |
| **Grip of ball on needle without locking:** | High level of grip, difficult to slide needle | Good grip - friction fit but easy to move | Good grip - friction fit but easy to move | Difficult to achieve reasonable friction fit |
| **Ability to rotate in socket when nut is disengaged:** | Not easily rotated in assembly | Easily rotated in assembly | Easily rotated in assembly | Easily rotated in assembly |
| **Ability to lock to needle when tightening nut is engaged:** | Tightly grips and locks needle | Tightly grips and locks needle | Tightly grips and locks needle | Does not grip needle |

From the results in Table 1 it is clear that a low durometer ball (Silicone, durometer: 2A) is effective at gripping the needle, but not fully suitable for this application due to its high degree of friction exerted by the ball on the socket that prevents the user from easily articulating the needle in the device. On the other hand, a higher durometer material (polyurethane, durometer 95A) can easily rotate within the socket but due to it's high stiffness cannot easily be tightened and locked to the needle. Of the devices tested in Table 1, optimal results were achieved for a 70A durometer ball with both Nitrile and Viton materials providing positive results.

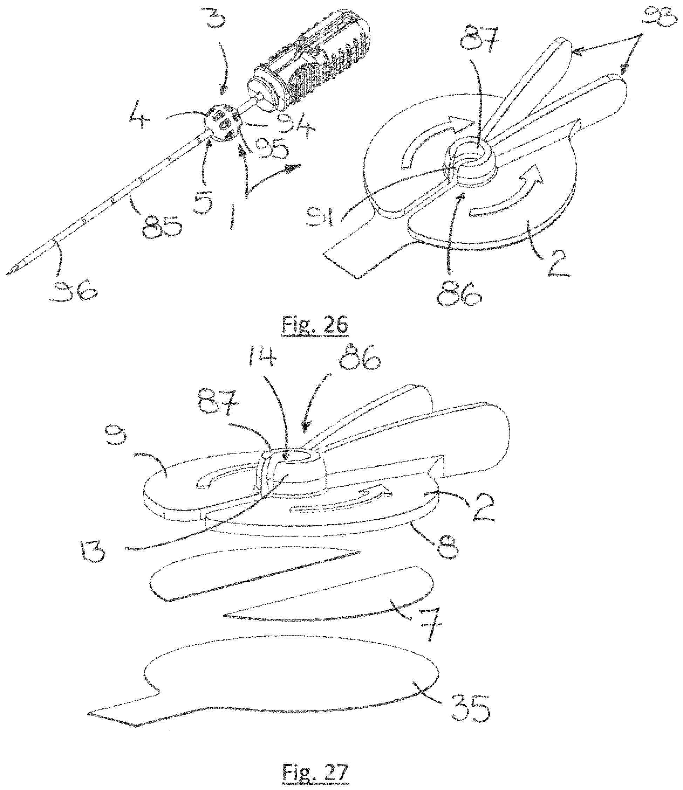

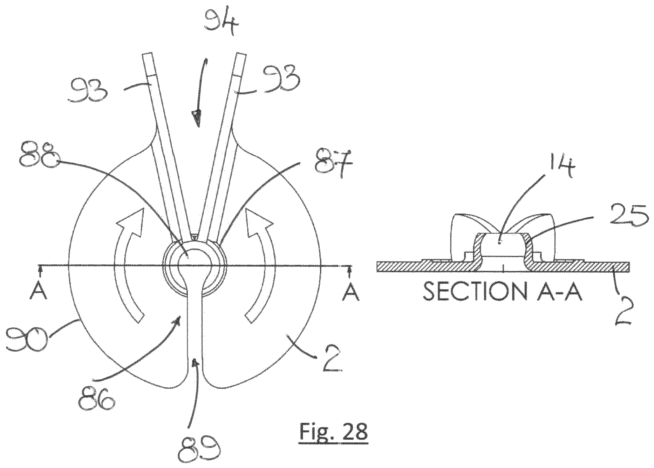

Figures 26 to 31 show an embodiment of the medical device stabilizer 1 of the invention similar in function to the medical device stabilizer 1 of Figures 1 to 25 and like numerals indicate like parts. However, in the present embodiment, the medical device stabilizer 1 is a two-part medical device stabilizer 1 made up of the base member 2 and the medical device grip 3 with the medical device grip 3 being separate/independent of the base member 2 to be slidably mountable on a medical device (which in the present embodiment is exemplified in the form of a coaxial delivery needle 85) without the base member 2, and being engageable with and detachable from the base member 2 at a coupler 86 in the form of a hinged clamping mechanism 87 and the coupler 86 is movable between a medical device grip engaged position and a medical device grip release position.

More particularly, as shown in the drawings, the medical device stabilizer 1 is made up of the base member 2 which is in the form of a base plate 2 and the medical device grip 3 which is in the form of a ball 4. As discussed in more detail below, the ball 4 can be detachably coupled to the base plate 2 at the coupler 86 formed on the base plate 2 which can be in the form of a clamping mechanism 87 as shown in the present embodiment.

The base plate 2 is a substantially circular or disc-like base plate 2 and has a base plate bottom face 8 and a base plate top face 9. The coupler 86 is centrally formed on the top face 9 and is made up of the clamping mechanism 87 which is formed by an adjustable socket 14 sized and shaped for receiving and holding the ball 4. The socket 14 is defined by a collet 13 centrally formed on the base plate 2. The collet 13 is integrally formed by the base plate 2 so that the collet 13 and base plate 2 constitute a unitary piece which can be injected moulded if desired.

The collet 13 and spherical socket 14 are sized to be complementary in size and shape with the ball 4 to receive and hold the ball 4. More particularly, the collet 13 is formed by a flexible collet wall 15 upstanding from the base plate 2. Internally, the collet wall 15 is shaped and spherically contoured as indicated by the reference numeral 25 so that the socket 14 complements the spherical shape of the ball 4.

In one embodiment of the present embodiment, the hinged clamping mechanism 87 is not a compression mechanism and does not exert a compressing force on the bore 5 of the medical device grip 3. In a second embodiment, the collet 13 and spherical socket 14 are undersized to the ball 4 and can be used to exert a clamping force on the bore 5 of the medical device grip 3.

The collet wall 15 is formed around a central opening 88 formed in the base plate 2 and a slit 89 extends from the central opening 88 to the edge 90 of the base plate 2 so that the collet wall 15 is not continuous i.e. is provided with a gap 91 contiguous with and defined by the slit 89. The socket 14 can therefore be opened and closed i.e. increased and decreased in size to receive and release the ball 4 by increasing and decreasing the size of the slit 89 and gap 91. To achieve this, the vicinity of the collet 13 serves as a hinge 92 so that the clamping mechanism 87 is a hinged clamping mechanism 87. More particularly, the collet wall 15 is provided with a pair of fingergrips 93 which can be pinched by a user to effect a ball clamping action. The fingergrips 93 are formed on the top face 9 of the base plate 2 and are spaced apart by a V-shaped indent 94 formed in the base plate 2. Accordingly, pinching together of the fingergrips 93 causes the socket 14 to hinge open to receive a ball 4 while release of the fingergrips causes the socket 14 to hinge closed to clamp the ball 4.

As indicated above, the medical device grip 3 is in the form of a ball 4 having a guide bore 5 therethrough for receiving and holding the medical devices in a slidable friction fit relationship. In one embodiment, the ball can be formed from a resilient material. The ball 4, and in particular the surface of the guide bore 5, is configured so that the ball 4 can maintain a constant friction-fit type contact with medical devices inserted in the guide bore 5. The ball 4 can be so configured by appropriate selection of the material of the ball e.g. a suitably resilient material or the material properties or structure of the surface defining the guide bore 5. Accordingly, when mounted on a medical device such as the co-axial needle 85, the ball 4 can be slid to a selected position on the co-axial needle 85 to mark the depth of insertion of the co-axial needle into a subject. The insertion depth can be indicated by depth markers 96 on the co-axial needle 85.

The outer surface 94 of the ball 4 can be provided with grip indents 95 to assist in manoeuvring the ball 4 and to assist in injection molding of the ball 4 by providing a uniform cross-sectional wall thickness throughout the ball 4.

The medical device stabilizer 1 can be secured to a patient via an adhesive e.g. in the form of an adhesive layer or tape 7 provided on the base plate 2 which is generally provided with a backing tab 35.

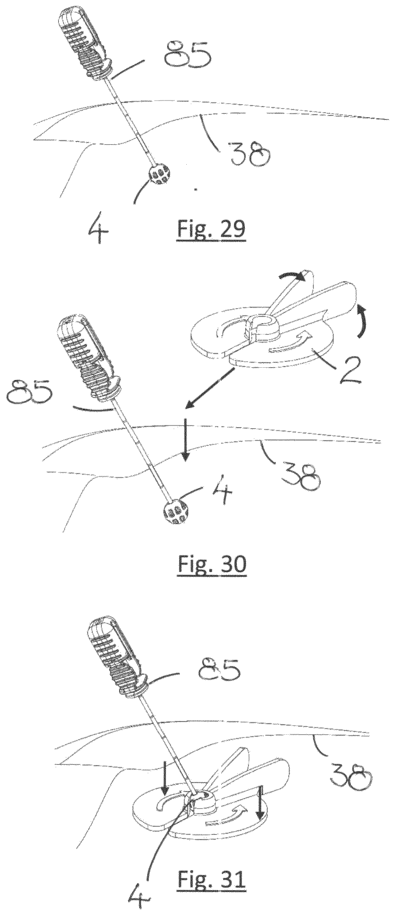

In use, the stabilizer device 1 can be used with a range of medical devices and in a variety of procedures such as biopsy, drainage, injection and ablation procedures. Figures 29 to 31 show the stabilizer device 1 in use with the CT-guided coaxial needle 85 which is first inserted through the guide bore 5 of the ball 4 so that the ball 4 is mounted on the CT-guided needle 85 in a frictionally slidable relationship i.e. the ball 4 can be slidably positioned as required on the co-axial needle 85 to select the desired insertion depth and remains in the desired position on the co-axial needle 85 due to the friction force - i.e. the guide bore 5 can be configured in size, structure and/or material so that medical devices enjoy a slidable friction fit in the guide bore 5 to hold the ball 4 on the CT-guided needle 85.

At the start of a CT-guided procedure, it may be necessary for the clinician to reposition the co-axial needle 85 to a preferred insertion position in the body. Having the ball 4 independent of the base member 2, allows the clinician greater flexibility in repositioning the co-axial needle 85 if required as the clinician does not have to attach and then detach the base member 2 at this stage. The base member 2 is engaged with the ball 4 when the preferred insertion position is confirmed under CT-guidance.

The CT-guided needle 85 is then inserted into a patient body part 38 (see Figure 29) under X-ray guidance and the CT-guided needle 85 can be positioned as required with the ball 4 fixed in place on the needle 85 under a frictional force, and, following removal of the removable backing tab 35 from the adhesive tape 7, the ball 4 is then clamped by the clamping mechanism 87 as previously described to hold the ball 4 and hence the needle 85 in the desired position (see Figures 30 and 31). The CT-guided needle 85 is therefore supported in place in the medical device stabilizer 1 with the clamping mechanism 87 preventing undesirable translational or sideways movement of the CT-guided needle 85 and the frictional sliding relationship between the bore 5 and the CT-guided needle 85 allowing controlled sliding movement of the needle as required.

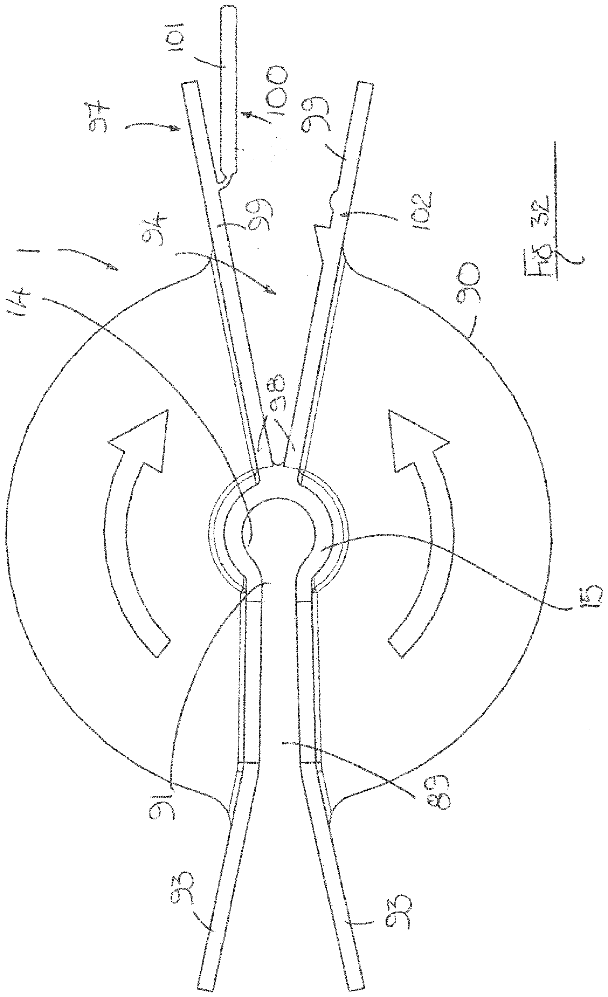

Figure 32 shows a top plan view of the base member 2 of a further embodiment of the stabilizer 1 of the invention in which the base member 2 of Figures 26 to 31 is also provided with a compression mechanism 97 to compress the ball 4, which in the present embodiment is a compressible ball 4, and firmly lock medical devices in position in the ball 4. Like numerals indicate like parts.

In the present embodiment, the medical device stabilizer 1 is employed in the same manner as the medical device of Figures 26 to 31 to stabilize medical devices. However, once stabilized, the medical devices can be fully locked in position by the compression mechanism 97 which is made up of a pair of compression hinges 98 formed on the top face 9 of the base plate 9 at the collet wall 15 and disposed opposite to the slit 89 and the gap 91. The compression hinges 98 can be hingedly opened and closed by second fingergrips 99 extending from the compression hinges 98 towards the base plate edge also with a V-shaped indent 94 therebetween formed in the base plate 2. In order to hold the compression mechanism 97 in place a locking mechanism 100 is provided on the base plate 2 in the form of a living hinge 100. The living hinge 100 is disposed between the second fingergrips 99 to be lockable in position to maintain the compression force at the socket 14. More particularly, the living hinge is made up of a finger 101 on one fingergrip 97 and a catch 102 on a second finger grip 97.

As will be appreciated by those skilled in the art, the present invention, and in particular in the embodiments shown in Figures 26 to 32, also extends to a medical device stabilizer system made up of the medical device stabilizer 1 and a medical device.

The stabilizer 1 of the invention can be formed from any suitable materials provided the material of the conformable or compressible medical device grip is sufficiently resilient to deform to provide a medical device gripping force in use. More particularly, the compressible and conformable resilient material of the compressible medical device grip should be sufficiently stiff to enable the compressible ball 4 to articulate and rotate and remain within the socket 14 using the snap-fit mechanism previously described whilst also being sufficiently resilient and compressible to grip the medical device when required. For example, the material of the compressible ball 4 should have a durometer reading of from about 10A to about 100A, more preferably from about 20A to about 90A and more preferably from about 40A to about 80A. During initial prototyping, a compressible ball 4 having a durometer reading of about 70A has been found to have a desirable resilience for articulation/rotation and compression of the compressible ball 4. Particularly suitable materials for the compressible ball include rubbers, nitriles, polyurethanes, neoprenes and silicones.

Nevertheless, where the medical device grip is not required to be compressible, it has also been found that a medical device grip formed from a resilient material is particularly suitable. For example, in this embodiment, a ball 4 formed from a resilient material having a durometer reading of from about 10A to about 100A, more preferably from about 20A to about 90A and more preferably from about 40A to about 80A is also suitable. A ball 4 having a durometer reading of about 70A has been found to have a desirable resilience for articulation/rotation. Again, particularly suitable materials for the compressible ball include rubbers, nitriles, polyurethanes, Pebax^{®}, neoprenes and silicones.

In a preferred embodiment the medical device grip 3 may be a formed from a ball 4 with a guide bore 5. However, in another example not forming part of the claimed invention its profile may also be ovoidal, tubular, or of any cross sectional shape containing a guide bore 5. The socket 14 of the base member 2 can be tailored to accept the profile medical device grip 3.

The base member 2 can also be formed from any suitable materials such as polymeric materials. In particular, the base member 2 of the embodiments shown in Figures 26 to 32, can be formed from polymeric materials exhibiting suitable mechanical properties e.g. sufficiently high yield strain characteristics to enable the hinged opening and closing of the socket 14 i.e. so that the hinge 92 can undergo repeated compression and extension forces to enable repeated opening and closing of the socket 14 in an analogous manner to a clothes peg. Examples of suitable materials are medical grade polymers such as acrylonitrile butadiene styrene (ABS), polyamide, polypropylene, polyethylene and polyether ketone (PEEK).

In other embodiments of the invention, the hinged clamping mechanism can be formed by and/or augmented with biasing means to create a biased clamping mechanism. The biased hinged clamping mechanism can be formed by the materials of the base member 2 or by mechanical means such as springs and the like. The effect of the biasing hinge clamping mechanism is to ensure that the socket 14 returns to its closed configuration after opening. The biasing hinge clamping mechanism may also be used to apply a clamping force on the medical device grip 3.

If desired, the stabilizer can be formed from transparent materials to allow for skin visualisation while various components can be colour coded in accordance with the size of the medical device to be used with the stabilizer e.g. needle gauges and can also be provided with inserts to adapt the guide bore to different needle gauges if desired.

Alternatively, due to the use of the compressive grip, the stabilizer can be a one size fits all universal stabilizer for all needle gauge sizes.

If desired, the stabilizer can be provided with other features such as a protractor to show the angle of orientation of medical devices gripped in the stabilizer.

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A medical device stabilizer (1) comprising
a base member (2);
a medical device grip (3) comprising a ball (4) mountable on a medical device (85) in a frictionally slidable relationship and positionable on the medical device (85) to select a desired medical device insertion depth, and defining a medical device guide bore (5) through the medical device stabilizer (1), and **characterised by**
a coupler (86) in the form of a hinged clamping mechanism (87) on the base member (2) for engaging the medical device grip (3), the coupler (86) being movable between a medical device grip engaged position and a medical device grip release position wherein the medical device stabilizer (1) is a two-part medical device stabilizer (1) in which the medical device grip (3) is separate and independent of the base member (2) but is engageable with and detachable from the base member (2) at the coupler (86) and wherein the medical device guide bore (5) is configured to form a slidable friction fit with a medical device (85) and hold the medical device grip (3) in a desired position on the medical device (85) without the base member (2) in use.

2. A medical device stabilizer (1) as claimed in Claim 1 wherein the medical device grip (3) comprises a resilient material.

3. A medical device stabilizer (1) as claimed in Claim 2 wherein the resilient material has a durometer reading of from about 20A to about 90A.

4. A medical device stabilizer (1) as claimed in Claim 3 wherein the resilient material has a durometer reading of from about 40A to about 80A.

5. A medical device stabilizer (1) as claimed in Claim 4 wherein the resilient material has a durometer reading of about 70A.

6. A medical device stabilizer (1) as claimed in any of Claims 1 to 5 wherein the clamping mechanism (87) comprises a collet (13) defining a socket (14) for the medical device grip (3).

7. A medical device stabilizer (1) as claimed in Claim 6 wherein the collet (13) is formed around a central opening (88) formed in the base member (2).

8. A medical device stabilizer (1) as claimed in Claim 7 wherein the collet (13) serves as a hinge (92) of the clamping mechanism (87).

9. A medical device stabilizer (1) as claimed in Claim 8 wherein the clamping mechanism (87) comprises at least one fingergrip (93) to effect a medical device grip (3) clamping action.

10. A medical device stabilizer (1) as claimed in Claim 1 wherein the clamping mechanism (87) comprises a pair of pinchable fingergrips (93) operable to hingedly open the socket (14) to receive a medical device grip (3) and hingedly close the socket (14) to clamp the medical device grip (3).

## Patentansprüche

1. Medizinvorrichtungsstabilisator (1), umfassend:
ein Basiselement (2);
einen Medizinvorrichtungshalter (3), umfassend eine Kugel (4), die in einer reibend gleitfähigen Beziehung an einer Medizinvorrichtung angebracht werden kann und an der Medizinvorrichtung (85) positionierbar ist, um eine gewünschte Einführtiefe der Medizinvorrichtung zu wählen, und die eine Medizinvorrichtungsführungsbohrung (5) durch den Medizinvorrichtungsstabilisator (1) definiert, und
**gekennzeichnet durch**
einen Koppler (86) in Form eines Gelenkeinspannmechanismus (87) auf dem Basiselement (2) zum Ineingriffnehmen des Medizinvorrichtungshalters (3), wobei der Koppler (86) zwischen einer Medizinvorrichtungshalter-Eingriffsstellung und einer Medizinvorrichtungshalter-Freigabestellung bewegbar ist, wobei der Medizinvorrichtungsstabilisator (1) ein zweiteiliger Medizinvorrichtungsstabilisator (1) ist, in dem der Medizinvorrichtungshalter (3) von dem Basiselement (2) getrennt und unabhängig ist aber an dem Koppler (86) mit dem Basiselement (2) in Eingriff gebracht und davon gelöst werden kann, und wobei die Medizinvorrichtungsführungsbohrung (5) dazu konfiguriert ist, eine gleitfähige Reibpassung mit einer Medizinvorrichtung (85) zu bilden und den Medizinvorrichtungshalter (3) im Gebrauch ohne das Basiselement (2) in einer gewünschten Stellung an der Medizinvorrichtung (85) zu halten.

2. Medizinvorrichtungsstabilisator (1) nach Anspruch 1, wobei der Medizinvorrichtungshalter (3) ein elastisches Material umfasst.

3. Medizinvorrichtungsstabilisator (1) nach Anspruch 2, wobei das elastische Material einen Durometerwert von etwa 20A bis etwa 90A aufweist.

4. Medizinvorrichtungsstabilisator (1) nach Anspruch 3, wobei das elastische Material einen Durometerwert von etwa 40A bis etwa 80A aufweist.

5. Medizinvorrichtungsstabilisator (1) nach Anspruch 4, wobei das elastische Material einen Durometerwert von etwa 70A aufweist.

6. Medizinvorrichtungsstabilisator (1) nach einem der Ansprüche 1 bis 5, wobei der Einspannmechanismus (87) ein Spannfutter (13) umfasst, das eine Aufnahme (14) für den Medizinvorrichtungshalter (3) definiert.

7. Medizinvorrichtungsstabilisator (1) nach Anspruch 6, wobei das Spannfutter (13) um eine in dem Basiselement (2) gebildete mittige Öffnung (88) gebildet ist.

8. Medizinvorrichtungsstabilisator (1) nach Anspruch 7, wobei das Spannfutter (13) als Gelenk (92) des Einspannmechanismus (87) dient.

9. Medizinvorrichtungsstabilisator (1) nach Anspruch 8, wobei der Einspannmechanismus (87) mindestens einen Fingergriff (93) zum Bewirken Einspannwirkung auf den Medizinvorrichtungshalter (3) umfasst.

10. Medizinvorrichtungsstabilisator (1) nach Anspruch 1, wobei der Einspannmechanismus (87) ein Paar quetschbarer Fingergriffe (93) umfasst, die betätigbar sind, um die Aufnahme (14) gelenkig zu öffnen, um einen Medizinvorrichtungshalter (3) aufzunehmen, und die Aufnahme (14) gelenkig zu schließen, um den Medizinvorrichtungshalter (3) einzuspannen.

## Revendications

1. Stabilisateur de dispositif médical (1) comprenant :
un élément de base (2) ;
un élément de préhension de dispositif médical (3) comprenant une boule (4) pouvant être montée sur un dispositif médical (85) dans une relation capable de coulisser par friction et d'être positionnée sur le dispositif médical (85) pour sélectionner une profondeur d'insertion de dispositif médical souhaitée, et définissant un alésage de guide de dispositif médical (5) à travers le stabilisateur de dispositif médical (1), et **caractérisé par**
un élément de couplage (86) sous la forme d'un mécanisme de serrage articulé (87) sur l'élément de base (2) destiné à entrer en prise avec l'élément de préhension de dispositif médical (3), l'élément de couplage étant capable de mouvement entre une position d'entrée en prise d'élément de préhension de dispositif médical et une position de libération d'élément de préhension de dispositif médical dans lequel le stabilisateur de dispositif médical (1) est un stabilisateur de dispositif médical (1) en deux parties dans lequel l'élément de préhension de dispositif médical (3) est distinct et indépendant de l'élément de base (2) mais est capable d'entrer en prise et de se détacher de l'élément de base (2) au niveau de l'élément de couplage (86) et dans lequel l'alésage de guide de dispositif médical (5) est configuré pour former un ajustement par friction capable de coulisser avec un dispositif médical (85) et maintenir l'élément de préhension de dispositif médical (3) dans une position souhaitée sur le dispositif médical (85) sans l'élément de base (2) en cours d'utilisation.

2. Stabilisateur de dispositif médical (1) selon la revendication 1, dans lequel l'élément de préhension de dispositif médical (3) comprend un matériau résiliant.

3. Stabilisateur de dispositif médical (1) selon la revendication 2, dans lequel le matériau résiliant a un relevé de duromètre allant d'environ 20A à environ 90A.

4. Stabilisateur de dispositif médical (1) selon la revendication 3, dans lequel le matériau résiliant a un relevé de duromètre allant d'environ 40A à environ 80A.

5. Stabilisateur de dispositif médical (1) selon la revendication 4, dans lequel le matériau résiliant a un relevé de duromètre d'environ 70A.

6. Stabilisateur de dispositif médical (1) selon l'une quelconque des revendications 1 à 5, dans lequel le mécanisme de serrage (87) comprend un collet (13) définissant un emboîtement (14) pour l'élément de préhension de dispositif médical (3).

7. Stabilisateur de dispositif médical (1) selon la revendication 6, dans lequel le collet (13) est formé autour d'une ouverture centrale (88) formée dans l'élément de base (2).

8. Stabilisateur de dispositif médical (1) selon la revendication 7, dans lequel le collet (13) sert d'articulation (92) du mécanisme de serrage (87).

9. Stabilisateur de dispositif médical (1) selon la revendication 8, dans lequel le mécanisme de serrage (87) comprend au moins un élément de préhension pour doigt (93) pour effectuer une action de serrage d'élément de préhension de dispositif médical (3).

10. Stabilisateur de dispositif médical (1) selon la revendication 1, dans lequel le mécanisme de serrage (87) comprend une paire d'éléments de préhension pour doigt (93) pouvant être pincés, capables de fonctionner pour ouvrir de manière articulée l'emboîtement (14) pour recevoir un élément de préhension de dispositif médical (3) et fermer de manière articulée l'emboîtement (14) pour serrer l'élément de préhension de dispositif médical (3).
